(19) Europäisches Patentamt / European Patent Office / Office européen des brevets

(11) **EP 2 305 322 B1**

(12) **EUROPÄISCHE PATENTSCHRIFT**

(45) Veröffentlichungstag und Bekanntmachung des Hinweises auf die Patenterteilung:
**19.09.2018 Patentblatt 2018/38**

(21) Anmeldenummer: **10005888.2**

(22) Anmeldetag: **08.06.2010**

(51) Int Cl.:
***A61B 17/34*** (2006.01) ***A61L 29/16*** (2006.01)
***A61L 31/16*** (2006.01) ***A61M 25/00*** (2006.01)
***A61B 17/00*** (2006.01) ***A61B 42/00*** (2016.01)
***A61B 46/00*** (2016.01) ***A61B 42/10*** (2016.01)

(54) **Polymerer medizinischer oder medizintechnischer oder PSA-Artikel mit antimikrobieller Ausrüstung**

Polymeric medicinal or medical or PSA item with antimicrobial features

Article médical polymérique, ou pour la technologie médicale, ou PSA doté d'un équipement antimicrobien

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.06.2009 DE 102009029762**

(43) Veröffentlichungstag der Anmeldung:
**06.04.2011 Patentblatt 2011/14**

(73) Patentinhaber: **Paul Hartmann Aktiengesellschaft 89522 Heidenheim (DE)**

(72) Erfinder: **Hietkamp, Peter 89518 Heidenheim (DE)**

(56) Entgegenhaltungen:
**WO-A2-2007/027871 WO-A2-2009/080239**
**US-A1- 2006 188 552**

- **M. K. OULE ET AL: "Polyhexamethylene guanidine hydrochloride-based disinfectant: a novel tool to fight meticillin-resistant Staphylococcus aureus and nosocomial infections", JOURNAL OF MEDICAL MICROBIOLOGY, Bd. 57, Nr. 12, 1. Dezember 2008 (2008-12-01), Seiten 1523-1528, XP055099201, ISSN: 0022-2615, DOI: 10.1099/jmm.0.2008/003350-0**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 305 322 B1

Printed by Jouve, 75001 PARIS (FR)

## Beschreibung

Die Erfindung betrifft medizinische oder medizintechnische polymere Artikel oder polymere Artikel für die persönliche Schutzausrüstung (PSA - Artikel) mit antimikrobieller Ausrüstung. Insbesondere betrifft die vorliegende Erfindung Handschuhe, insbesondere Untersuchungshandschuhe und/oder Operationshandschuhe und/oder Schutzhandschuhe, Katheter, insbesondere Blasenkatheter und/oder Venenkatheter, Komponenten für ein Drainage-Systeme, insbesondere eines intraoperativen Drainage-Systems und/oder eines postoperativen Drainage-Systems, Kanülen, Schläuche mit Einsatz im medizinischen Umfeld, oder sonstige für den medizinisch invasiven Einsatz vorgesehene polymere Artikel, Auffangbeutel, und auch Folien, wie Abdeck-/Schutz-/Verpackungsfolien für den Einsatz im medizinischen Umfeld umfassend ein polymeres Trägermaterial oder bestehend aus einem polymeren Trägermaterial, welches eine antimikrobielle Ausrüstung aufweist.

Die Problematik von Infektionen, auch von Infektionstransfer vor allem in Krankenhäusern und in sonstigen medizinischen Einrichtungen ist bekannt. So ist die nosokomiale Infektion ein großes Problem. Unter nosokomialer Infektion wird die durch Mikroorganismen hervorgerufene Infektion verstanden, die im Zusammenhang mit einem Aufenthalt in einer medizinischen Einrichtungen und Krankenhaus besteht. Faktoren sind oftmals Patienten mit bereits geschwächter körpereigener Infektionsabwehr und invasive medizinische Massnahmen, welche ein erhöhtes Infektionsrisiko mit sich bringen. Zudem bringt u.a. ein vermehrter und nicht sachgemäßer Einsatz von Antibiotika in der Behandlung von bakteriellen Infektionen auch noch das Problem der Zunahme von multiresistenten Keimen mit sich.

Infektionsherde sind dabei insbesondere Wundinfektionen nach Operationen, HarnwegsInfektionen, Atemwegsinfektionen und Sepsis (Übertritt des Erregers in die Blutbahn). Die Erreger gelangen insbesondere bei chirurgischen Eingriffen oder bei Anwendung von invasiven Hilfsmitteln in den Körper. Im Rahmen von invasiven medizinischen Maßnahmen werden also in den Patienten körperfremde Komponenten eingeführt, zumeist auch mit einer längeren Verweildauer. Invasive Hilfsmittel sind dabei beispielsweise Venenkatheter (möglicherweise Auslöser für nosokomiale Septikämien), Harnwegskatheter (möglicherweise Auslöser nosokomialer Harnwegsinfektionen), Intubation und Beatmungsschläuche für apparative Beatmung (möglicherweise Auslöser von Beatmungspneumonien) oder Liquorableitungen (möglicherweise Auslöser für Meningitiden).
Wesentlicher Ausgangspunkt für die Pathogenese von derartigen Infektionen ist oftmals zunächst die Anheftung von Mikroorganismen und damit potentiellen Infektionserregern an die im medizinischen Umfeld eingesetzten Hilfsmittel, wie beispielsweise an das Plastikmaterial eines Katheters. An der Übergangstelle zum Körperinneren bzw. dann im Körperinneren wachsen die Keime weiter heran.

Infektionen können endogen bedingt sein, also durch die eigene Patientenflora an Mikroorganismen verursacht sein oder auch exogener Art sein, also durch nicht standortübliche Erreger durch Übertragung verursacht sein.

Präventionsmaßnahmen zur Vermeidung von Infektionen und insbesondere von Infektionstransfer sind ein ganz wesentlicher Bestandteil des alltäglichen medizinischen Arbeitsablaufes:
So hilft die Händedesinfektion Infektionen zu vermeiden, und damit einhergehend die Entstehung von nosokomialen Infektionen oder auch multiresistenten Keimen zu unterdrücken.
Die Händedesinfektion ist eine Präventionsmaßnahme, die gemäß den 5 Indikationen entsprechend den Richtlinien der WHO und des Robert-Koch-Instituts erfolgen soll:

1) Grundsätzlich vor jedem Patientenkontakt, und dabei auch vor dem Anlegen der Handschuhe;
2) unmittelbar vor aseptischen Tätigkeiten, wie beispielsweise bei Kontakt mit invasiven Hilfsmitteln (wie Katheter), bei Kontakt mit nicht intakter Haut wie beim Anlegen von Verbänden und Injektionen, bei Schleimhautkontakt;
3) nach Kontakt mit potentiell infektiösem Material, wie beispielsweise bei Kontakt mit nicht intakter Haut (Anlegen von Verbänden, bei Kontakt mit invasiven Hilfsmitteln (Blutabnahme über Katheter, Wechsel von Sekretbeuteln));
4) nach Patientenkontakt, dabei insbesondere auch nach dem Ausziehen der Handschuhe; und 5) nach Verlassen der unmittelbaren Patientenumgebung, auch ohne direkten Kontakt zum Patienten gehabt zu haben.

Die Durchführung der Händedesinfektion - hygienisch oder chirurgisch - und deren Wirksamkeit wird gemäß den Referenzverfahren EN 1500 bzw. EN 12791 untersucht und festgelegt. Trotz Vorgaben hinsichtlich der Einreibemethode für die Händedesinfektion ist die Problematik der möglicherweise verbleibenden Benetzungslücken bei Einreibung des Händedesinfektionsmittels bekannt.

Auch Handschuhe sind ein wichtiger Schutz für den Träger, wie also dem medizinischen Personal. Handschuhe vermindern die Keimlast an den Händen des Personals, jedoch verhindern Handschuhe nicht die Übertragung, weshalb auch nach Handschuhgebrauch eine Händedesinfektion durchzuführen ist.

Auch für medizinische Instrumente ist eine entsprechende Methodik der Desinfektion vorgegeben.

Die Bereitstellung und Herstellung von antimikrobiellen Bedingungen in einer jeweiligen Situation durch eine entsprechende erforderliche in situ -Anwendung von antimikrobiellen Wirksubstanzen, wie durch direktes Besprühen, Einreiben, Abwischen ist bekannt und wird auch durchgeführt.

Der Nachteil dabei ist, dass die Ausführung des Desinfektionsschritts oftmals in einer Notfallsituation und damit auch unter zeitlichem Druck durchgeführt werden muss. So können insbesondere trotz der bekannten Standard-Einreibemethoden wie vorangehend dargestellt bei der Händedesinfektion Benetzungslücken des Desinfektionsmittels an den Händen bzw. auf den Handschuhen verbleiben.

Antimikrobiell ausgerüstete Gegenstände sind in der Medizin und in der Medizintechnik schon bekannt. Hierbei soll die antimikrobielle Ausrüstung sowohl den Gegenstand bzw. das Material aus dem der Gegenstand besteht vor dem Befall durch Mikroorganismen schützen oder zumindest die Vermehrung von Mikroorganismen in oder auf dem Gegenstand unterdrücken, als auch die unmittelbare Umgebung des Artikels vor dem Befall durch Mikroorganismen bewahren oder zumindest die Vermehrung von Mikroorganismen in dieser Umgebung unterdrücken. Dabei werden unterschiedlichste antimikrobielle Wirkstoffe (Mikrobizide) zum Einsatz gebracht. Die eingesetzten Mikrobizide sind Stoffe unterschiedlicher chemischer Zusammensetzung, die Mikroorganismen wie beispielsweise Viren, Bakterien, Pilze oder Algen abtöten können. Daher werden Mikrobizide u.a. auch nach ihrer Wirksamkeit unterschieden in Antibiotika, Konservierungsmittel, Desinfektionsmittel, Schleimbekämpfungsmittel und Anstrichfungizide oder auch nach ihrer spezifischen Wirkung gegen verschiedene Mikroorganismengruppen unterschieden in Algizide, Bakterizide, Fungizide und Viruzide. Wirkstoffe, die die Vegetation von Mikroorganismen nur hemmen, ohne sie abzutöten, bezeichnet man auch als Mikrobiostatika wie beispielsweise Bakteriostatika oder Virostatika. Über die chemische Struktur und die Eigenschaften der bekannten Mikrobizide besitzt man meist genaue Kenntnisse. Dagegen ist bezüglich ihrer Wirkungsorte in den Zellen und über die in den Zellen auf molekularer Ebene ablaufenden Wirkungsmechanismen wenig bekannt. Nichtsdestotrotz kann man eine große Anzahl von Mikrobiziden nach ihrem Wirkungsmechanismus charakterisieren als membranaktive oder elektrophilaktive Wirkstoffe. Eine eindeutige Zuordnung ist jedoch nicht immer möglich.

Zu den membranaktiven Mikrobiziden zählen beispielsweise: Alkohole, Säuren, Phenole, Salicylanilide, Carbanilide, Dibenzamidine, Wirkstoffe mit kationogenem Charakter, wie quartäre Ammonium-Verbindungen, Biguanide und Azol-Fungizide. Den membranaktiven Wirkstoffen ist gemeinsam, dass sie die Zellmembran adsorptiv belegen können. Vor allem in Gegenwart nicht letaler Mikrobizidkonzentrationen ist dies zunächst ein reversibler Vorgang, der jedoch zu Veränderungen an der äußeren Membran und der Zellwand führt. Die äußeren Barrieren verlieren ihre Integrität, wodurch der Zugang zur Cytoplasmamembran für die Mikrobizidmoleküle ermöglicht wird und somit die letalen Effekte ausgelöst werden können.

Aus der Anmeldung WO 2009/080239 A2 ist der Einsatz von Polyhexamethylenguanidin oder ein Salz davon für die antimikrobielle Ausstattung von textilen Trägermaterialien von medizinischen oder medizintechnischen Artikeln bekannt.

WO 2007/027871 A2 beschäftigt sich mit der Ausstattung von im medizinischen Umfeld einsetzbaren Artikeln mit PHMB (Polyhexamethylenbiguanidin).

In der Abhandlung M.K. Oulé et al, Journal of Medical Microbiology, (2008), 57, 1523-1528 wird die antimikrobielle Wirkung von PHMGH (Polyhexamethylenguanidinhydrochlorid) anhand von Untersuchungsergebnissen beschrieben.

Aufgabe der vorliegenden Erfindung ist es nun, medizinische und medizintechnische Artikel oder Artikel für die persönliche Schutzausrüstung umfassend ein polymeres Trägermaterial oder bestehend aus einem polymeren Trägermaterial mit unspezifisch aber dennoch hoch wirksamen antimikrobiellen Ausrüstungen zur Verfügung zu stellen, die die Besiedelung durch pathogene Keime auf den Artikeln und in der näheren Umgebung der Artikel effektiv hemmen oder verhindern, also sowohl die weitere Kolonisation als auch die Übertragung hemmen oder vermeiden. Hierbei soll insbesondere sichergestellt sein, dass weder der Anwender noch ein Patient durch den Umgang mit dem Produkt in seiner Gesundheit beeinträchtigt wird. Durch diese Ausrüstung soll weithin sichergestellt sein, dass auch das Wachstum von denjenigen Keimen effektiv gehemmt oder gehindert wird, die gegen bereits eingesetzte antimikrobiell wirkende Mittel Resistenzen aufgebaut haben.

Gelöst wird die Aufgabe durch einen medizinischen oder medizintechnischen Artikel oder Artikel für die persönliche Schutzausrüstung umfassend ein polymeres Trägermaterial oder bestehend aus einem Trägermaterial mit einer ersten oberflächenbildenden Seite und einer zweiten oberflächenbildenden Seite, welches herstellerseitig mit einer antimikrobiellen Zubereitung ausgerüstet ist mit den Merkmalen nach Anspruch 1.

Dabei weist Polyhexamethylenguanidin gemäß der vorliegenden Erfindung die folgende chemische Struktur gemäß Formel (I) auf:

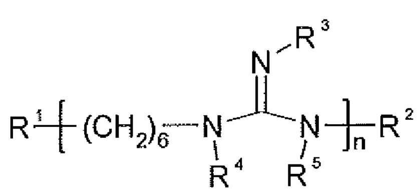

(I)

wobei gilt: n = 10 bis 80
$R^1$ = H, OH, $NH_2$ oder NH-C(=NH)-$NH_2$;
$R^2$, $R^3$, $R^4$, $R^5$ = gleichzeitig oder unabhängig voneinander H, OH, $(CH_2)_6$-$NH_2$ oder $(CH_2)_6$-OH.

Bevorzugt bedeutet gemäß Formel (I) gleichzeitig oder unabhängig voneinander:

n = 10 bis 80
$R^1$ = H, $NH_2$ oder NH-C(=NH)-$NH_2$;
$R^2$ = H oder $(CH_2)_6$-$NH_2$;
$R^3$, $R^4$, $R^5$ = H.

Weiterhin bevorzugt kann n = 20 bis 80, insbesondere n = 30 bis 80, insbesondere n = 30 bis 70 oder besonders bevorzugt n ± 30 bis 60 bedeuten. Es kann jedoch auch vorgesehen sein, dass n = 35 bis 80, insbesondere n = 35 bis 70, insbesondere n = 35 bis 60, insbesondere n = 40 bis 80 oder besonders bevorzugt n = 40 bis 70 bedeutet.

Weiterhin kann das Polyhexamethylenguanidin gemäß der vorliegenden Erfindung auch als Kation insbesondere als Polykation gemäß der folgenden Formel (II) vorliegen:

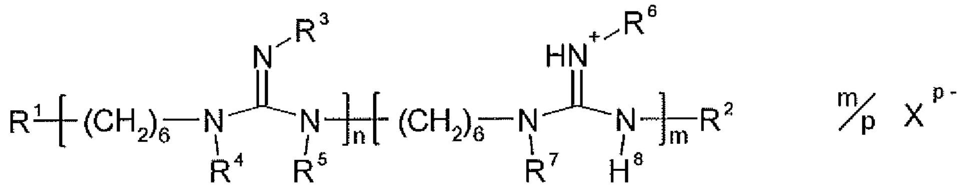
(II)

wobei gilt: m = 1 bis 80 und n = 0 bis 79, wobei $10 \leq n + m \leq 80$ gilt;
$R^1$ = H, OH, $NH_2$, $NH_2^+$, NH-C(=NH)-$NH_2$ oder NH-C(=$NH_2^+$)-$NH_2$;
$R^2$ ,$R^3$, $R^4$, $R^5$ = gleichzeitig oder unabhängig voneinander H, OH, $(CH_2)_6$-$NH_2$, $(CH_2)_6$-OH oder $(CH_2)_6$-$NH_2^+$;
$R^6$, $R^7$, $R^8$= gleichzeitig oder unabhängig voneinander H, $(CH_2)_6$-$NH_2$, $(CH_2)_6$-OH oder $(CH_2)_6$-$NH_2^+$;
p = 1, 2 oder 3; und
X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

In einer bevorzugten Ausführung der Erfindung bedeutet in Formel (II) gleichzeitig oder unabhängig voneinander:

m = 1 bis 80 und n = 0 bis 79, wobei $10 \leq n + m \leq 80$ gilt;
$R^1$ = H, $NH_2$, $NH_2^+$, NH-C(=NH)-$NH_2$ oder NH-C(=$NH_2^+$)-$NH_2$;
$R^2$ ,$R^3$, $R^4$, $R^5$ = gleichzeitig oder unabhängig voneinander H, $(CH_2)_6$-$NH_2$ oder $(CH_2)_6$-$NH_2^+$;
$R^6$, $R^7$, $R^8$ = gleichzeitig oder unabhängig voneinander H, $(CH_2)_6$-$NH_2$, oder $(CH_2)_6$-$NH_2^+$;
p = 1, 2 oder 3; und
X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

In einer weiteren bevorzugten Ausführung der Erfindung bedeutet in Formel (II) gleichzeitig oder unabhängig voneinander:

m = 1 bis 80 und n = 0 bis 79, wobei $10 \leq n + m \leq 80$ gilt;
$R^1$ = H, $NH_2^+$, NH-C(=NH2$^+$)-$NH_2$, $NH_2$ oder NH-C(=$NH_2$)-$NH_2$;
$R^2$ = H, $(CH_2)_6$-$NH_2^+$ oder $(CH_2)_6$-$NH_2$;
$R^3$, $R^4$, $R^5$, $R^6$, $R^7$, $R^8$=H;
p = 1, 2 oder 3; und

X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

Weiterhin bevorzugt kann gemäß Formel (II) der vorliegenden Erfindung insbesondere $20 \leq n + m \leq 80$, insbesondere $30 \leq n + m \leq 80$, insbesondere $30 \leq n + m \leq 70$ und besonders bevorzugt $30 \leq n + m \leq 60$ bedeuten. Es kann jedoch auch vorgesehen sein, dass $35 \leq n + m \leq 80$, insbesondere $35 \leq n + m \leq 70$, insbesondere $30 \leq n + m \leq 60$, insbesondere $40 \leq n + m \leq 80$ und besonders bevorzugt $40 \leq n + m \leq 70$ bedeutet. Gleichzeitig oder unabhängig hiervon kann gemäß Formel (II) der vorliegenden Erfindung insbesondere m = 10 bis 80, insbesondere m = 20 bis 80, insbesondere m = 30 bis 80, insbesondere m = 30 bis 70 oder besonders bevorzugt m = 30 bis 60 und n = 0 bis 79 bedeuten. Es kann jedoch auch vorgesehen sein, dass m = 5 bis 80, insbesondere m = 15 bis 80, insbesondere m = 25 bis 80, insbesondere m = 35 bis 80, insbesondere m = 40 bis 80, insbesondere m = 35 bis 70, insbesondere m = 35 bis 60, insbesondere m = 40 bis 80, insbesondere m = 40 bis 70 und n = 0 bis 60 bedeutet.

Damit kann weiterhin bevorzugt das Polyhexamethylenguanidin gemäß der vorliegenden Erfindung auch als Salz vorliegen. Falls das Polyhexamethylenguanidin als Salz vorliegt, so liegt das Grundpolymer als Kation oder Polykation vor, dessen positive Ladungen durch einwertige, zweiwertige oder dreiwertige anorganische oder organische Anionen ausgeglichen sind. Insbesondere sind als Anionen Chlorid, Bromid, Nitrat, Phosphat, Hydrogenphosphat, Dihydrogenphosphat, Citrat, Hydrogencitrat, Dihydrogencitrat, Sulfat, Hydrogensulfat, Carbonat, Hydrogencarbonat, Acetat, Gluconat, Lactat oder Mischungen hiervon geeignet. Besonders bevorzugt ist Chlorid, Phosphat und Hydrogenphosphat.

Ganz besonders bevorzugt umfasst die Zubereitung Polyhexamethylenguanidiniumhydrochlorid (CAS 57 028-96-3).

Polyhexamethylenguanidin oder ein Salz hiervon, insbesondere Polyhexamethylenguanidiniumhydrochlorid weist ein breites antimikrobielles Wirkspektrum auf. Polyhexamethylenguanidin oder ein Salz hiervon ist beispielsweise wirksam gegen Bakterien wie Echerichia coli, Pseudomonas aeruginosa, Staphylococcus aureus, Salmonella typhimurium, Salmonella gallinarium, Mycobacterium tuberculosis, Clostridium difficile, Coryme bacterium diphteriae, Shigella sonnei, Shigella flexneri, Proteus vulgaris, Pasteurella gallinarium oder Listeria monocitogenes. Zudem ist es wirksam gegen zahlreiche Pilze und Viren. Polyhexamethylenguanidin oder ein Salz hiervon, insbesondere Polyhexamethylenguanidiniumhydrochlorid ist auch antibakteriell wirksam gegen Bakterien, die bereits Resistenzen aufgebaut haben, wie beispielsweise Staphylococcus aureus (MRSA). Polyhexamethylenguanidin oder ein Salz hiervon hat katiogenen Charakter oder ist positiv geladen und kann damit gut mit den negativ geladenen Zellwänden der Keime reagieren, also eine elektrostatische Bindung mit den negativen geladenen Teilen der Zellwand eingehen. Die Semipermeabilität der Zellmembran wird gestört oder auch aufgelöst. Die Zerstörung der intakten Zellstruktur führt schließlich zum Zelltod der Keime. Diese Wirkungsweise basiert vielmehr auf einer physikalischen Wirkungsweise. Dadurch unterliegt Polyhexamethylenguanidin oder dessen Salze nicht der so großen Gefahr der Ausbildung von Resistenzen der Keime gegenüber Polyhexamethylenguanidin oder dessen Salze.

Vorteilhaft wird durch die antimikrobielle Ausrüstung von Artikeln mit polymerem Trägermaterial, ausgerüstet mit Polyhexamethylenguanidin oder dessen Salze eine zusätzliche Präventionsmaßnahme vor Infektionen und Infektionsübertragung geschaffen.

Aufgrund des polymeren Charakters ist Polyhexamethylenguanidin oder dessen Salze gegenüber bekannten niedermolekularen antimikrobiellen Verbindungen wie beispielsweise niedermolekulare quaternäre Stickstoffverbindungen z.B. Chlorhexidin oder Benzalkoniumchlorid wenig toxisch und weist eine hohe Stabilität gegen Ultraviolette Strahlung auf. Aufgrund des polymeren Charakters kann Polyhexamethylenguanidin oder dessen Salze nicht über die Haut in den Körper eines Menschen gelangen. Somit kann ein mit Polyhexamethylenguanidin oder dessen Salz ausgerüsteter Gegenstand direkt auf der Haut getragen werden.

Zudem sind Polyhexamethylenguanidin oder dessen Salze bislang auch als nicht Allergie auslösender Stoff bekannt. Zudem ist aus Akkumulationstests, ausgeführt in Tieren, bekannt, dass Polyhexamethylenguanidin oder dessen Salze keinerlei Rückstände in den Organen oder Muskulatur der Tiere zurücklässt. Polyhexamethylenguanidin, insbesondere in der Ausführung als Polyhexamethylenguanidiniumhydrochlorid (CAS 57 028-96-3) ist in der EU-Direktive, Annex II, als sicheres Produkt aufgenommen worden und damit auch für die Anwendungsbereiche, wie u.a. menschliche Hygiene, Desinfektionsmittel für den privaten Bereich und auch im öffentlichen Gesundheitswesen zugelassen.

Zudem wirkt Polyhexamethylenguanidin oder dessen Salz gegenüber einer Vielzahl an Materialien, wie insbesondere Metalle, Latex, Holz oder Plastikmaterialien nicht korrosiv, also nicht beschädigend.

Überraschender Weise hat sich zudem gezeigt, dass Polyhexamethylenguanidin oder dessen Salz davon auf einem polymeren Trägermaterial über einen längeren Zeitraum antibakteriell wirkt. Damit ist gemäß der vorliegenden Erfindung auch ein medizinischer oder medizintechnischer Artikel oder ein Artikel für die persönliche Schutzausrüstung ein bevorzugter Gegenstand, der ab dem erstmaligen Gebrauch mindestens 2 Tage, vorzugsweise mindestens 3 Tage, weiter vorzugsweise mindestens 4 Tage antimikrobiell wirksam ist.

Gemäß der vorliegenden Erfindung sind medizinische oder medizintechnische Artikel oder Artikel für die persönliche Schutzausrüstung ein Handschuh, insbesondere ein Untersuchungshandschuh und/oder ein Operationshandschuh und/oder ein Schutzhandschuh, ein Katheter, insbesondere ein Blasenkatheter und/oder ein Venenkatheter, eine Komponente für ein Drainage-System, insbesondere eines intraoperativen Drainage-Systems und/oder eines postoperativen Drainage-Systems, eine Kanüle oder ein Schlauch mit Einsatz im medizinischen Umfeld, sowie sonstige medizinisch invasive Hilfsmittel; ein Auffangbeutel und/oder eine Folie, wie insbesondere Abdeck-/Schutz-/Verpackungsfolien für den Einsatz im medizinischen Umfeld.

Handschuhe sollen den Patienten und den Anwender vor Kontaminationen, insbesondere als Untersuchungshandschuhe bei der Durchführung von medizinischen Untersuchungen schützen. In der Chirurgie eingesetzte Operationshandschuhe sind dabei sterile, anatomisch geformte medizinische Handschuhe. Schutz beim Umgang mit kontaminierten medizinischen Materialien oder für sonstige diagnostische oder therapeutische Zwecke sollen allgemein jegliche Schutzhandschuhe bieten. Untersuchungs- und Pflege/Schutzhandschuhe können steril oder unsteril sein, und können auch anatomisch geformt sein. Besonders bevorzugt handelt es sich bei den vorgenannten Handschuhen um wegwerfbare Handschuhe, also für den einmaligen Gebrauch vorgesehene Handschuhe. Bereits der Einsatz von wegwerfbaren Handschuhen unterstützt die Verminderung von Infektionstransfer.

Unter Drainage wird im medizinischen Umfeld die Ableitung /Absaugung von krankhaften und/oder vermehrt anfallenden natürlichen Körperflüssigkeiten verstanden. Dabei wird auch unterschieden zwischen innerer und äußerer Drainage. Bei der inneren Drainage werden operativ oder minimal invasiv eine Art Verbindung zwischen den Hohlorganen, wie Speiseröhre, Magen, Darm angelegt, um dann aufgestaute Flüssigkeiten in die entsprechenden Hohlorgane abzuleiten.

Bei der äußeren Drainage wird die Körperflüssigkeit aus dem Körperinneren nach außen abgeleitet. Die äußere Drainage wird im medizinischen Bereich sehr unterschiedlich und zumeist auch wenig standardisiert unterteilt. Die Unterteilung kann erfolgen beispielsweise nach dem Erfinder der jeweiligen Drainage-Methode, oder nach der Drainage unterzogenen Körperregion (z.B. Bauchdrainage), nach dem Krankheitszustand (bspw. Wunddrainage, Pneumothoraxdrainage) oder auch der Funktionsweise der Drainage (Schwerkraftdrainage, (Vakuum)saugdrainage, Kapillardrainage) oder auch dem Zeitpunkt des Einsatzes, wie insbesondere als Absaugung während einer Operation (intraoperative Drainage) oder nach der Operation (postoperative Drainage), so in Form einer Wunddrainage.

Bei der Durchführung einer jeglichen Drainage werden entsprechende Komponenten des Drainage-Systems eingesetzt. Diese Komponente unfassen Handstücke, Absaugschläuche, Verbindungsschläuche, alle zumeist aus polymeren Materialien. Die Komponenten der Drainage müssen insbesondere bei einer postoperativ durchzuführenden Drainage, also der Wunddrainage, in oder an der entsprechenden Körperregion/Wunde fixiert werden.

Ein bevorzugter Gegenstand der vorliegenden Erfindung sind insbesondere auch Handschuhe, insbesondere Untersuchungshandschuhe und/oder Operationshandschuhe und/oder Schutzhandschuhe, Katheter, insbesondere ein Blasenkatheter und/oder ein Venenkatheter, Komponenten für ein Drainage-System, insbesondere eines intraoperativen Drainage-Systems und/oder eines postoperativen Drainage-Systems, Kanülen, Schläuche mit Einsatz im medizinischen Umfeld, oder sonstige invasiven Hilfsmittel, Auffangbeutel, und auch Folien, wie Abdeck-/Schutz-/Verpackungsfolien für den Einsatz im medizinischen Umfeld umfassend jeweils ein polymeres Trägermaterial oder bestehend aus einem polymeren Trägermaterial, welches herstellerseitig mit einer antimikrobiellen Zubereitung ausgerüstet ist und wobei die Zubereitung Polyhexamethylenguanidin oder ein Satz hiervon, insbesondere Polyhexamethylenguanidiniumchlorid als antimikrobielles Mittel und insbesondere weitere Hilfs- oder Zusatzstoffe umfasst.

Als polymeres Trägermaterial für den medizinischen oder medizintechnischen Artikel oder den Artikel für die persönliche Schutzausrüstung können dabei verschiedene Materialien eingesetzt werden, wie insbesondere synthetische Homo- und Copolymere auf Basis von Polyolefinen, wie Polyethylen (HDPE und LDPE), Polypropylen, Polyisobutylen, Polybutadien, Polyisopren; Polymere und Copolymere aus vinylaromatischen Monomeren, wie Polystyrol, Poly(styrol-co-acrylnitril), Poly(styrol-co-butadien-co-acrylnitril) (ABS-Copolymere); halogenhaltige Polymere, wie Polyvinylchlorid, Polyvinylidenchlorid, Polychloropren, Polytetrafluorethylen (PTFE) und Polyvinylidenfluorid; Polykondensate, beispielsweise Polyester, wie Polyethylenterephthalat, Polybutylenterephthalat; Polyamide, wie Polycaprolactam, Polykondensate aus Hexamethylendiamin und Adipinsaeure; Polyacrylate und Polymethacrylate, wie Polymethylmethacrylat; Polycarbonate, Polyether, Polyetherketone, Polyacrylnitril, Polyurethane, Polyimide und Silikone. Ebenso können als polymeres Trägermaterial auch natürliche Polymere wie Naturkautschuk eingesetzt werden. Ebenso können auch Kombinationen der genannten synthetischen und/oder natürlichen Polymere eingesetzt werden.

Das polymere Trägermaterial kann dabei auch elastomere Eigenschaften aufweisen, insbesondere als polymeres Trägermaterial für die Handschuhe.

Als polymeres Trägermaterial für Handschuhe werden die für den Fachmann herkömmlich bekannten polymeren Materialien eingesetzt. Bekannt sind Naturkautschuk und/oder Synthesekautschuke oder Kombinationen davon. Die Synthesekautschuke sind vorzugsweise entnommen aus der Gruppe umfassend Isoprenkautschuk (synthetisches

Polyisopren), Chloropren-Kautschuk (Polychloropren), Acrylnitril-Butadien-Kautschuk (Nitrilkautschuk), hydrierter Nitrilkautschuk, Styrol-Butadien-Kautschuk, S-EB-S (Styrol-Ethylen-Buten-Styrol) Block-Copolymere, S-I-S (Styrol-Isoprene-Styrol) Block-Copolymere, S-B-S (Styrol-Butadien-Styrol) Block-Copolymere, S-I (Styrol-Isopren) Block-copolymere, S-B (Styrol-Butadien) Block-Copolymere, Butadien-Polymere, Styrol-Butadien Polymere, Carboxylierte Styrol-Butadien-Polymere, Acrylnitril-Styrol-Butadien Polymere, und deren Derivate.

Insbesondere vorzugsweise ist das polymere Trägermaterial des Handschuhs, insbesondere des Untersuchungshandschuhs und/oder des Operationshandschuhs und/oder des Schutzhandschuhs aus den polymeren Materialien entnommen aus der Gruppe Naturkautschuk und synthetischer Kautschuk, davon insbesondere synthetisches Polyisopren (Isoprenkautschuk), Polychloropren, Acrylonitril-Butadien-Kautschuk und Derivate, oder Kombinationen davon.

Als polymeres Trägermaterial für Folien werden vorzugsweise polymere Materialien entnommen aus der Gruppe Polyolefine, wie insbesondere Polyethylen und/oder Polypropylen und Polyvinylchlorid, oder Kombinationen davon eingesetzt.

Als polymeres Trägermaterial für die Komponenten für Drainage-Systeme werden insbesondere PVC (Polyvinylchlorid), Polyurethan, Silikon oder PU-Silikone und/oder Kombinationen eingesetzt.

Die antimikrobielle Ausrüstung des polymeren Trägermaterials erfolgt derart, dass das Polyhexamethylenguanidin oder ein Salz hiervon, insbesondere

Polyhexamethylenguanidiniumchlorid direkt der Masse zur Herstellung des polymeren Trägermaterials zugesetzt wird, d.h dass somit das Polyhexamethylenguanidin oder ein Salz hiervon, insbesondere Polyhexamethylenguanidiniumchlorid direkt in das polymere Trägermaterial integriert ist.

Polyhexamethylenguanidin oder ein Salz hiervon wird als Additiv direkt der Polymermatrixzusammensetzung, aus welcher das polymere Trägermaterial gebildet wird, zugesetzt. Aufgrund der hohen Temperaturresistenz der Molekülstruktur von Polyhexamethylenguanidin oder ein Salz hiervon, insbesondere Polyhexamethylenguanidiniumchlorid, von bis ca. 250°C ist diese Vorgehensweise möglich, ohne dass negative Veränderungen der Eigenschaften des antimikrobiellen Wirkstoffs zu befürchten sind. Der inline-Verarbeitungsprozess kann vorteilhafterweise zumeist mit weniger technischen Aufwand vorgenommen werden als ein entsprechender Ausrüstungsschritt auf dem fertigen polymeren Trägermaterial zu einem späteren Zeitpunkt. Insbesondere vorteilhaft wird hierbei ein polymeres Trägermaterial bereitgestellt, das durchgehend mit dem antimikrobiellen Wirkstoff versetzt ist, so dass der Wirkstoff damit auch auf der ersten und der zweiten oberflächenbildenden Seite des polymeren Trägermaterials bereitgestellt wird. Dies ist insbesondere vorteilhaft, da damit ein polymeres Trägermaterial mit einer antimikrobiellen Ausrüstung unanhängig vom späteren Einsatzbereich bzw. insbesondere unabhängig von der späteren für den Einsatz besonders bevorzugten ersten oder zweiten oberflächenbildenden Seite des Trägermaterials bereitgestellt wird. Es wird zudem vorteilhafterweise eine länger anhaltende Wirksamkeit gewährleistet; da vorzugsweise der antibakterielle Wirkstoff allmählich freigesetzt werden kann bzw. an die erste und/oder zweite oberflächenbildende Seite diffundieren kann. Polyhexamethylenguanidin oder ein Salz davon sind dabei insbesondere in der Polymermatrixzusammensetzung in einem Anteil von 0,02 - 2,0 Gew-%, weiter insbesondere von 0,05 - 1,5 Gew-%, weiter insbesondere von 0,07 - 1,2 Gew-% enthalten. Polyhexamethylenguanidin oder ein Salz davon kann dabei in Form einer antimikrobiellen Zubereitung mit einem höheren Anteil, insbesondere von 0,1 bis 50 Gew.-%, vorzugsweise 0,2 bis 45 Gew.-%, weiter vorzugsweise 1 bis 40 Gew.-%, weiter insbesondere 5 bis 35 Gew.-%, weiter inbesondere von 10 - 30 Gew-% zugeben werden.

Gemäß einer besonders bevorzugten Ausführung der vorliegenden Erfindung kann die Zubereitung als Hilfs- oder Zusatzstoff insbesondere ein Hautpflegemittel, ein Tensid, ein Hydrophobisierungsmittel, ein Hydrophilisierungsmittel, ein Gleitmittel, ein Netzmittel und/oder ein Avivagemittel umfassen. Hierbei ist insbesondere vorgesehen, dass eine Menge an Hilfs- oder Zusatzstoff bezogen auf die Oberfläche des polymeren Trägermaterials 0,0001 bis 150 mg/ $cm^2$ beträgt. Insbesondere weist das polymere Trägermaterial einen Gehalt von 0,1 bis 900 $\mu$g/ $cm^2$, insbesondere 0,1 bis 500 $\mu$g/ $cm^2$, insbesondere 0,1 bis 300 $\mu$g/ $cm^2$ und ganz besonders bevorzugt 0,1 bis 150 $\mu$g/ $cm^2$ bezogen auf die Fläche des Trägermaterials auf.

Insbesondere weist die Zubereitung kein Wasser auf. Somit ist der erfindungsgemäße Artikel insbesondere bis auf einen durch vorhandene Luftfeuchtigkeit verursachten Restwassergehalt als wasserfrei zu bezeichnen.

Vorzugsweise umfasst die Zubereitung als Hautpflegemittel insbesondere einen Pflanzenextrakt, wobei der Pflanzenextrakt beispielsweise durch Extraktion der gesamten Pflanze, aber auch ausschließlich durch Extraktion aus Blüten, Blättern, Samen, Wurzeln und/oder anderen Pflanzenteilen, hergestellt werden kann. Erfindungsgemäß sind vor allem die Extrakte aus speziellen Pflanzen wie Grünem Tee, Hamamelis, Kamille, Stiefmütterchen, Aloe Vera, Rosskastanie, Salbei, Weidenrinde, Chrysanthemen, Eichenrinde, Brennnessel, Hopfen, Klettenwurzel, Schachtelhalm, Weissdorn, Lindenblüten, Mandeln, Fichtennadeln, Sandelholz, Wacholder, Kokosnuss, Kiwi, Guave, Limette, Mango, Aprikose, Weizen, Melone, Orange, Grapefruit, Avocado, Rosmarin, Birke, Buchensprossen, Wiesenschaumkraut, Schafgarbe, Quendel, Thymian, Melisse, Eibisch, Veilchen, Blättern der Schwarzen Johannisbeere, Huflattich, Fünffingerkraut, Ginseng, Ingwerwurzel und Süßkartoffel als Pflanzenextrakt bevorzugt.

Insbesondere umfasst die Zubereitung einen Pflanzenextrakt aus Grünem Tee, Hamamelis, Ringelblume oder

Aloe Vera. Die erfindungsgemäßen Zubereitungen können auch Mischungen aus mehreren, insbesondere aus zwei, verschiedenen Pflanzenextrakten enthalten. In einer besonders bevorzugten Ausführungsform ist in der erfindungsgemäßen Zubereitung Aloe Vera enthalten.

Insbesondere vorzugsweise ist bei Handschuhen, insbesondere bei Untersuchungshandschuhen, Operationshandschuhen, und/oder Schutzhandschuhen vorgesehen, dass die antimikrobielle Zubereitung Hautpflegemittel umfasst. Vorzugsweise umfasst die Zubereitung als Tensid insbesondere ein nichtionisches Tensid, ein kationisches Tensid, ein anionisches Tensid oder ein amphoteres Tensid.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-14-Alkohole mit 3 EO bis 7 EO, C9-11-Alkohol mit 7 EO, C13-15-Alkohole mit 3 EO, 5 EO, 7 EO oder 8 EO, C12-18-Alkohole mit 3 EO, 5 EO oder 7 EO und Mischungen aus diesen, wie Mischungen aus C12-14-Alkohol mit 3 EO und C12-18-Alkohol mit 7 EO. Die angegebenen Ethoxylierungsgrade stellen statistische Mittelwerte dar, die für ein spezielles Produkt eine ganze oder eine gebrochene Zahl sein können. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise sondern statistisch verteilt sind. Solche Produkte sind durch gleichzeitige Einwirkung von Ethylenoxid (EO) und Propylenoxid (PO) auf Fettalkohole erhältlich.

Beispiele für Fettalkoholpolyethylenglycolether sind solche mit der Formel (III)

$$R^1O\text{-}(CH_2CH_2O)_n\text{-}H \qquad \text{(III)}$$

wobei für gilt: n = 1 bis 5;
$R^1$ = eine linearer oder verzweigter Alkyl- und/oder Alkenylrest mit 6 bis 22, vorzugsweise 12 bis 18 Kohlenstoffatomen.

Als kationische Tenside können insbesondere quartäre Ammoniumverbindungen, kationische Polymere und Emulgatoren, wie in Mitteln zur Textilavivage eingesetzt werden. Geeignete Beispiele für quartäre Ammoniumverbindungen sind Stoffe der Formeln (IV) und (V):

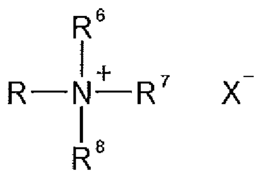

(IV)

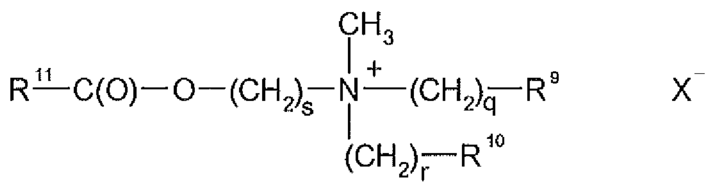

(V)

wobei in Formel (IV) gilt:

R, $R^6$ = ein acyclischer Alkylrest mit 12 bis 24 Kohlenstoffatomen;
$R^7$ = ein gesättigter C1-C4 Alkyl- oder Hydroxyalkylrest;
$R^8$ = entweder gleich R, Ra oder Rb ist oder für einen aromatischen Rest steht;
$X^-$ = ein Halogenid-, Methosulfat-, Methophosphat- oder Phosphatanion sowie Mischungen hiervon.

Verbindungen der Formel (V) sind sogenannte Esterquats. Hierfür gilt:

$R^9$ = H, OH oder O(CO)-$R^{12}$, wobei $R^{12}$ unabhängig von $R^{11}$ und $R^{13}$ für einen aliphatischen Alkylrest mit 12 bis 22 Kohlenstoffatomen oder für einen aliphatischen Hydroxyalkylrest mit 12 bis 22 Kohlenstoffatomen oder für einen

aliphatischen Alkenylrest mit 12 bis 22 Kohlenstoffatomen mit 1, 2 oder 3 Doppelbindungen steht;
$R^{10}$ = H, OH oder O(CO)-$R^{13}$, wobei $R^{13}$ unabhängig von $R^{12}$ und $R^{11}$ für einen aliphatischen Alkylrest mit 12 bis 22 Kohlenstoffatomen oder für einen aliphatischen Hydroxyalkylrest mit 12 bis 22 Kohlenstoffatomen oder für einen aliphatischen Alkenylrest mit 12 bis 22 Kohlenstoffatomen mit 1, 2 oder 3 Doppelbindungen steht;
$R^{11}$ = unabhängig von $R^{12}$ und $R^{13}$ ein aliphatischer Alkylrest mit 12 bis 22 Kohlenstoffatomen oder ein aliphatischen Hydroxyalkylrest mit 12 bis 22 Kohlenstoffatomen oder ein aliphatischer Alkenylrest mit 12 bis 22 Kohlenstoffatomen mit 1, 2 oder 3 Doppelbindungen;
q, r, s = jeweils unabhängig voneinander 0, 1, 2, 3, 4 oder 5, wobei jedoch $q + r + s \geq 1$ gilt;
$X^-$ = Halogenid-, Methosulfat-, Methophosphat- oder Phosphation sowie Mischungen hiervon.

Für bevorzugte Verbindungen gemäß Formel V gilt:

$R^9$ = O(CO)-$R^{12}$, wobei $R^{12}$ unabhängig von $R^{11}$ und $R^{13}$ für einen aliphatischen Alkylrest mit 16 bis 18 Kohlenstoffatomen oder für einen aliphatischen Alkenylrest mit 16 bis 18 Kohlenstoffatomen mit 1, 2 oder 3 Doppelbindungen steht;
$R^{10}$ = O(CO)-$R^{13}$, wobei $R^{13}$ unabhängig von $R^{11}$ und $R^{13}$ für einen aliphatischen Alkylrest mit 16 bis 18 Kohlenstoffatomen oder für einen aliphatischen Alkenylrest mit 16 bis 18 Kohlenstoffatomen mit 1, 2 oder 3 Doppelbindungen steht;
$R^{11}$ = unabhängig von $R^{12}$ und $R^{13}$ ein aliphatischer Alkylrest mit 16 bis 18 Kohlenstoffatomen oder ein aliphatischer Alkenylrest mit 16 bis 18 Kohlenstoffatomen mit 1, 2 oder 3 Doppelbindungen;
q, r, s = jeweils unabhängig voneinander 0, 1, 2 oder 3, wobei jedoch $q + r + s \geq 1$ gilt;
$X^-$ = Halogenid-, Methosulfat-, Methophosphat- oder Phosphation sowie Mischungen hiervon.

Beispiele für kationische Verbindungen der Formel (IV) sind insbesondere Didecyldimethylammoniumchlorid, Ditalgdimethylammoniumchlorid oder Dihexadecylammoniumchlorid.

Beispiele für Verbindungen der Formel (V) sind Methyl-N-(2-hydroxyethyl)-N,N-di(talgacyloxyethyl)ammoniummethosulfat, Bis-(palmitoyl)-ethyl-hydroxyethyl-methylammoniummethosulfat oder Methyl-N,N-bis(acyloxyethyl)-N-(2-hydroxyethyl)ammoniummethosulfat.

Um die Benetzbarkeit des polymeren Trägermaterials zu verbessern und somit auch die antimikrobielle Wirksamkeit zu verbessern, kann die Zubereitung insbesondere auch ein Hydrophilierungsmittel umfassen. Beispiele für derartige Hydrophilierungsmittel sind ein- oder mehrwertigen Alkohole, Alkanolamine oder Glycolether, sofern sie mit Wasser mischbar sind. Vorzugsweise werden die Hydrophilierungsmittel ausgewählt Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder - propyl-ether, Dipropylenglykolmonomethyl-, oder - ethylether, Diisopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether, Alkoholen, insbesondere C1-C4-Alkanole, Glykole, Polyethylenglykole, vorzugsweise mit einem Molekulargewicht zwischen 100 und 100000, insbesondere zwischen 200 und 10000, und Polyolen, wie Sorbitol und Mannitol, sowie bei Raumtemperatur flüssiges oder feste Polyethylenglykol, Carbonsäureestern, Polyvinylalkoholen, Ethylenoxid-Propylenoxid-Blockcopolymeren sowie beliebigen Gemischen der voranstehenden.

Insbesondere weist ein erfindungsgemäßer medizinischer oder medizintechnischer Artikel oder Artikel für persönliche Schutzausrüstung, ein Handschuh, insbesondere ein Untersuchungshandschuh und/oder ein Operationshandschuh und/oder ein Schutzhandschuh, ein Katheter, insbesondere ein Blasenkatheter und/oder ein Venenkatheter, eine Komponenten für ein Drainage-System, insbesondere eines intraoperativen Drainage-Systems und/oder eines postoperativen Drainage-Systems, eine Kanüle oder ein Schlauch mit Einsatz im medizinischen Umfeld, ein Auffangbeutel und/oder eine Folie, wie insbesondere Abdeck-/Schutz-/Verpackungsfolien für den Einsatz im medizinischen Umfeld eine erste oberflächenbildende Seite auf, die durch die erste oberflächenbildende Seite des polymeren Trägermaterials gebildet wird. Diese erste oberflächenbildende Seite des Artikels kann im anwendungsgerechten Zustand des Artikels zum Patienten oder zum Anwender gerichtet sein. Es kann jedoch auch vorgesehen sein, dass diese erste oberflächenbildende Seite des Artikels vom Patienten oder vom Anwender wegweist.

So ist insbesondere auch vorgesehen, dass die erste oberflächenbildende Seite des polymeren Trägermaterials eine Außenfläche des Artikels bildet.
Weiter vorzugsweise bildet die zweite oberflächenbildende Seite des polymeren Trägermaterials eine Innenseite des Artikels.
Insbesondere bevorzugt bilden die erste und die zweite oberflächenbildende Seite des polymeren Trägermaterials die entsprechende Außenseite und Innenseite des Artikels.

Im Rahmen dieser Erfindung ist die "Außenseite" eines Artikels, insbesondere bei einem Artikel mit einer Formgebung, die Oberseite, welche von dem durch die Formgebung zumindest teilweise umschlossenen Raum abgewandt ist. So ist im Falle von Handschuhen die Außenseite die Oberseite des Handschuhs, welche im für den Gebrauch vorgesehenen und im für den Anwender bereitgestellten Zustand und auch im angezogenen Zustand des Handschuhs eine dem Körper des Anwenders abgewandte Lage ist.

"Außen" bzw. "Außenseite" bedeutet grundsätzlich eine vom Rauminneren des Artikels und/oder vom Körper des Anwenders abgewandte Orientierung.

Die "Innenseite" ist dem entsprechend die Oberseite des Artikels, welche dem durch die Formgebung des Artikels zumindest teilweise umschlossenen Raum am nächsten ist. So ist im Falle von Handschuhen die Innenseite die Oberseite, welche im für den Gebrauch vorgesehenen und im für den Anwender bereitgestellten Zustand und auch im angezogenen Zustand des Handschuhes dem Körper des Anwenders zugewandt ist. "Innen" bzw. "Innenseite" bedeutet grundsätzlich eine dem Rauminneren eines Artikels und/oder dem Körper des Anwenders zugewandte Orientierung.

Generell ist die Ausrüstung der Außenseite des Handschuhs, insbesondere des Untersuchungshandschuhs und/oder des Operationshandschuhs und/oder des Schutzhandschuhs eine vorteilhafte Maßnahme, um das Risiko der Übertragung von Keimen zu minimieren. Im Falle einer Operation wird der als spätere Operationsfeld bildende Bereich und auch der an das Operationsfeld angrenzende Bereich auf der Haut des Patienten entsprechend desinfiziert vorbereitet; dennoch besteht die Gefahr, dass Erreger aus der Keimflora des Patienten durch Hantieren des Chirurgen über die Handschuhe in das Operationsfeld und damit in die offene Wunde eingebracht werden. Insbesondere auch bei Arbeiten in einem an sich unsterilen Umfeld, wie beispielsweise bei der Patientenwundversorgung wie Verbandswechsel, Setzen von Kanülen, Anlegen von Infusionen vermindert die den antimikrobiellen Wirkstoff aufweisende Außenseite des Handschuhs, insbesondere des Untersuchungshandschuhs, die Gefahr der Keimverschleppung.

Generell ist die Ausrüstung der Innenseite des Handschuhs, insbesondere des Untersuchungshandschuhs und/oder des Operationshandschuhs und/oder des Schutzhandschuhs eine vorteilhafte Maßnahme, um die Gefahr der Übertragung der Keimflora des Trägers auf sein Umfeld, wie u.a. auch auf den Patienten, und auch beispielsweise in das Operationsfeld oder in die Patientenumgebung u.a. nach dem Ausziehen oder beim Wechsel der Handschuhe zu vermindern.

Ein Gegenstand der Erfindung ist ein Handschuh, insbesondere ein Untersuchungshandschuh und/oder ein Operationshandschuh und/oder ein Schutzhandschuh, bei dem die antimikrobielle Zubereitung in dem polymeren Trägermaterial integriert ist. Insbesondere vorzugsweise ist der Handschuh, insbesondere der Untersuchungshandschuh und/oder der Operationshandschuh und/oder der Schutzhandschuh aus dem polymeren Trägermaterial gebildet.

Ein weiterer Gegenstand der Erfindung ist eine Komponente eines Drainage-Systems und/ oder eine Kanüle und/oder ein Schlauch mit Einsatz im medizinischen Umfeld und/oder ein Katheter, insbesondere ein Blasenkatheter und/oder ein Venenkatheter ist, wobei bei diesen Artikeln die antimikrobielle Zubereitung in das polymere Trägermaterial integriert ist, wobei insbesondere diese eine Komponente eines Drainage-Systems und/ oder diese Kanüle und/oder dieser Schlauch mit Einsatz im medizinischen Umfeld und/oder dieser Katheter, insbesondere dieser Blasenkatheter und/oder dieser Venenkatheter weiter aus dem polymeren Trägermaterial gebildet sind.

Hervorzuheben ist an dieser Stelle, dass die hier aufgeführten Merkmale der alternativen oder bevorzugten Ausgestaltungen der Erfindung nicht auf die einzelnen Alternativen oder Bevorzugungen zu beschränken sind. Im Zusammenhang mit der vorliegenden Erfindung ist es vielmehr der Fall, dass eine Kombination von Ausgestaltungen bzw. eine Kombination jedes Einzelmerkmals einer alternativen Form mit Merkmalen einer anderen alternativen Ausgestaltung ebenso als erfindungsgemäßer Gegenstand zu sehen ist. Ebenso wenig soll die Erfindung durch die nachfolgende Beschreibung der Zeichnungen reduziert verstanden sein.

Die Figur 1 zeigt einen Handschuh, insbesondere einen Untersuchungshandschuh, einen Operationshandschuh und/oder einen Schutzhandschuh. Der in Figur 1 dargestellte Handschuh 10 besteht aus einem polymeren Trägermatenal 12, welches eine erste Oberseite 14 und eine zweite Oberseite 16 aufweist. Die erste oberflächenbildende Seite 14 bildet dabei die Außenseite 15 des Handschuhs, also die dem Träger abgewandte Oberseite und eben bei Patientenkontakt die dem Patienten zugewandte Oberseite. Die zweite oberflächenbildende Seite 16 bildet die Innenseite 17 des Handschuhs, also die dem Träger zugewandte oberflächenbildende Seite.

In nicht erfindungsgemäßer Weise wird die erste oberflächenbildende Seite 14 des polymeren Trägermaterials 12, und damit die Außenseite 15 des Handschuhs 10 mit einer 10 - 30 Gew.-% wässrigen Polyhexamethylenguanidiniumchlorid-Lösung (CAS 57 028-96-3) besprüht, derart, dass auf der Außenseite 15) des Handschuhs (10) der Wirkstoff bezogen auf die Fläche in einer Menge von 10 bis 1750 $\mu g/ cm^2$ und ganz besonders bevorzugt 10 bis 1500 $\mu g/ cm^2$, ganz besonders bevorzugt von 100- 1000 $\mu g/ cm^2$ vorhanden ist.

Anschließend wird der Handschuh trocknen gelassen.

Prüfung auf antimikrobielle Aktivität

Das Prüfverfahren dient zur quantitativen Bestimmung der Wirkung der antimikrobiellen Ausrüstung bei den polymeren Materialien. Dazu wird das polymere Material mit dem jeweiligen Bakterienstamm, der zuvor in Weichagar gegeben wird, beimpft. Nach definierten Zeiten werden Keimzahlbestimmungen durchgeführt. Das Prüfverfahren wird

angelehnt an die ASTM E 2180-01 durchführt.
Als mikrobielle Indikatoren werden vorzugsweise die Bakterienstämme: Staphylococcus aureus ATCC 6538 (DSM 346), Klebsiella pneumoniae ATCC 4352 (DSM 789) eingesetzt.
Für den Versuch werden benötigt:

- Sterile Schere, sterile Pinzette
- Stanzeisen mit Durchmesser 4 cm
- Sterile Flaschen, Petrischalen, Reagenzgläser
- Brutschrank (37°C ± 2 °C)
- Ringerlösung mit Enthemmern (0,3% Lecithin, 0,1% Histidin, 1% Tween). Die Ringerlösung wird durch Auflösen von 1 Ringer-Tablette (erhältlich bei der Fa. Merck unter der Materialnummer 1.15525.0001) in 500 ml destilliertem Wasser hergestellt. Eine Ringertablette enthält 0,00525 g Ammoniumchlorid, 0,005 g Natriumhydrogencarbonat, 0,04 g Calciumchlorid - 2 Hydrat, 0,00525 g Kaliumchlorid und 1,125 g Natriumchlorid.
- CASO-Agar = Caseinpepton-Sojamehlpepton-Agar (erhältlich bei der Fa. Merck unter der Materialnummer 1.05458.0500)
- CASO-Bouillon (erhältlich bei der Fa. Merck unter der Materialnummer 1.05459.0500)
- 0,85%-ige Kochsalzlösung
- Weichagar (hergestellt aus Mischung von 0,85 g Kochsalz, 0,3 g Agar- Agar (erhältlich bei Fa. Merck unter der Materialnummer 1.01614.1000) und 100 ml destilliertes Wasser)
- Bakterienstämme: Staphylococcus aureus ATCC 6538 (DSM 346), Klebsiella pneumoniae ATCC 4352 (DSM 789)

Vorbereitung:

Vom polymeren Material werden Prüfstücke mit einer definierten Größe/Dimension ausgestanzt bzw. geschnitten. Die Anzahl der Prüfstücke wird so gewählt, dass mindestens eine Dreifachbestimmung zu den definierten Zeiten nach der Beimpfung möglich ist.
Unter Prüfstücke werden dabei die mit antimikrobiellem Wirkstoff ausgerüsteten Probenmaterialstücke sowie die Kontrollmaterialstücke ohne antimikrobielle Wirkstoffausrüstung verstanden.
Die Prüfstücke werden unter den für das Prüfstück geeigneten Bedingungen sterilisiert. Für den Versuch wird der jeweilige Bakterienstamm mit Kolonien von 1-5 x $10^8$ KBE/ml (KBE = Kolonie bildende Einheiten) eingesetzt.

Versuchsdurchführung:

Bei dem gesamten Prüfverfahren wird die eingesetzte Keimzahl zusätzlich überprüft, d.h. sämtliche Prüfverfahrensschritte werden ohne Einbringen eines Prüfstücks durchgeführt (Keimzahl-Kontrolle).
Jedes Prüfstück (also Proben - und Kontrollmaterialstück) wird in eine Petrischale gegeben. Mittels eines sterilen Tupfers, welcher mit einer sterilen 85 %-igen Kochsalzlösung getränkt ist, wird jedes Prüfstück durch Abtupfen befeuchtet. Diese Befeuchtung gewährleistet eine gleichmäßige Verteilung des nachfolgend aufzubringenden Weichagars.
1 ml der Bakterienkultur mit 1-5 x $10^8$ KBE/ml wird in 100 ml des auf 45 ± 2 °C eingestellten Weichagars gegeben. Damit liegen 1 - 5 x $10^6$ KBE/ml Bakterienkultur im Weichagar vor. Anschließend wird 1 ml des beimpften Weichagars vorsichtig auf das Prüfstück aufgebracht, derart dass ein dünner Film auf dem gesamten Prüfstück entsteht. Nach Erstarren des Weichagars werden die mit den Prüfstücken beladenen Petrischalen bis zum gewünschten Prüfzeitpunkt zur Keimzahlbestimmung bei 37 ± 2 °C in den Brutschrank gestellt.
Als Prüfzeitpunkte können beispielsweise Zeit 1= 0 h (also sofort, direkt nach Beladung des Prüfstücks mit beimpften Weichagar), Zeit 2= 4 h, Zeit 3 = 24 h oder noch spätere Zeitpunkte gewählt werden.
Zur Keimzahlbestimmung werden die Prüfstücke mittels 100 ml Ringerlösung mit Enthemmer jeweils in eine sterile 250 ml Schraubverschlussflasche gegeben. Zum Ablösen des Weichagars vom Prüfstück wird das Schraubverschlussgefäß für 1 min im Ultraschallbad behandelt und anschließend für 1 min mechanisch geschüttelt. Nachfolgend wird daraus eine Verdünnungsreihe angelegt, indem jeweils 1 ml aus der vorangehenden Lösung in ein steriles Röhrchen mit vorgelegten 9 ml an Ringerlösung mit Enthemmer eingebracht wird. Die Verdünnungsreihen werden derart angelegt, so dass eine spätere Auszählung der herangewachsenen Kolonien möglich ist. Bei Ansätzen mit zu erwartendem höheren Bakterienwachstum, wie den Kontrollmaterialstücken und den Keimzahl-Kontrollen, sind demnach höhere Verdünnungen anzusetzen.
Anschließend wird mit einer sterilen Pipette aus jeder Verdünnungsstufe 1 ml entnommen und in eine sterile Petrischale pipettiert und mit 15 - 20 ml Agarmedium (CASO-Agar) beschichtet. Zur gleichmäßigen Verteilung des Mediums werden die befüllten Petrischalen kreisend über die Arbeitsfläche bewegt. Nach Erstarren des Agars werden die Petrischalen mit dem Deckel nach unten in den Brutschrank gestellt und 18 - 24 Stunden bei 37 ± 2°C im Brutschrank bebrütet.
Die Auswertung der Keimzahlen erfolgt dann mittels Abzählen der gewachsenen Keimkolonien. Bei stärkerem Kolonienwachstum sind die Platten mit Kolonien zwischen 30 und 300 zur Auswertung heranzuziehen und möglichst die Zählwerte zweier Verdünnungsstufen zu berücksichtigen.
Die mittlere geschätzte Kolonienzahl ermittelt sich wie folgt:

$$x_m = \frac{\text{Summe Koloniezählwert}}{\text{Summe Verdünnungsstufen}}$$

$$\text{bspw. } x_m = \frac{303+290+32+28}{10^{-4}+10^{-4}+10^{-5}+10^{-5}} = \frac{653}{2{,}2\text{x}10^{-4}} = 2{,}97 \text{ x}10^{6}$$

Die ermittelten Werte werden dann in der Einheit KBE/g oder KBE/ml angegeben.

## Patentansprüche

1. Medizinischer oder medizintechnischer Artikel oder Artikel für die persönliche Schutzausrüstung (10) umfassend ein polymeres Trägermaterial (12) oder bestehend aus einem polymeren Trägermaterial (12) mit einer ersten oberflächenbildenden Seite (14) und einer zweiten oberflächenbildenden Seite (16), welches herstellerseitig mit einer antimikrobiellen Zubereitung ausgerüstet ist, **dadurch gekennzeichnet, dass** die Zubereitung Polyhexamethylenguanidin oder ein Salz hiervon als antimikrobielles Mittel umfasst, dass der Artikel ein Handschuh, insbesondere ein Untersuchungshandschuh und/oder ein Operationshandschuh und/oder ein Schutzhandschuh, ein Katheter, insbesondere ein Blasenkatheter und/oder ein Venenkatheter, eine Komponente für ein Drainage-System, insbesondere eines intraoperativen Drainage-Systems und/oder eines postoperativen Drainage-Systems, wobei die Komponente für ein Drainage-System, Handstücke, Absaugschläuche und Verbindungsschläuche, alle zumeist aus polymeren Materialien, umfassen eine Kanüle oder ein Schlauch mit Einsatz im medizinischen Umfeld, ein Auffangbeutel und/oder eine Folie, insbesondere eine Abdeck-/Schutz-/Verpackungsfolie für den Einsatz im medizinischen Umfeld ist, und dass die antimikrobielle Zubereitung in das polymere Trägermaterial integriert ist.

2. Medizinischer oder medizintechnischer Artikel oder Artikel für die persönliche Schutzausrüstung gemäß Anspruch 1, **dadurch gekennzeichnet, dass** die Zubereitung Polyhexamethylenguanidin oder ein Salz hiervon als antimikrobielles Mittel und weitere Hilfs- oder Zusatzstoffe umfasst.

3. Medizinischer oder medizintechnischer Artikel oder Artikel für die persönliche Schutzausrüstung gemäß Anspruch 2, **dadurch gekennzeichnet, dass** die Zubereitung als Hilfs- oder Zusatzstoff ein Hautpflegemittel, ein Tensid, ein Hydrophobisierungsmittel, ein Hydrophilisierungsmittel, ein Gleitmittel, ein Netzmittel und/ oder ein Avivagemittel umfasst.

4. Medizinischer oder medizintechnischer Artikel oder Artikel für die persönliche Schutzausrüstung gemäß mindestens einem der vorgenannten Ansprüche, **dadurch gekennzeichnet, dass** der Artikel ab dem erstmaligen Gebrauch mindestens 2 Tage, vorzugsweise mindestens 3 Tage, weiter vorzugsweise mindestens 4 Tage antimikrobiell wirksam ist.

## Claims

1. Medical or medical-technology article or article for personal protective equipment (10) comprising a polymeric support material (12) or consisting of a polymeric support material (12) having a first surface-forming side (14) and a second surface-forming side (16), which material has been provided with an antimicrobial preparation by the manufacturer, **characterized in that** the preparation comprises polyhexamethylene guanidine or a salt thereof as antimicrobial agent, **in that** the article is a glove, more particularly an examination glove and/or a surgical glove and/or a protective glove, a catheter, more particularly a urinary catheter and/or a venous catheter, a component for a drainage system, more particularly an inter-operative drainage system and/or a post-operative drainage system, the component for a drainage system encompassing hand pieces, suction tubes and connection tubes, all usually composed of polymeric materials, a cannula or a tube having use in the medical field, a collection bag and/or a film, more particularly a cover/protective/packaging film for use in the medical field, and **in that** the antimicrobial preparation has been integrated into the polymeric support material.

**2.** Medical or medical-technology article or article for personal protective equipment according to Claim 1, **characterized in that** the preparation comprises polyhexamethylene guanidine or a salt thereof as antimicrobial agent and further auxiliaries or additives.

**3.** Medical or medical-technology article or article for personal protective equipment according to Claim 2, **characterized in that** the preparation comprises, as auxiliary or additive, a skin-care agent, a surfactant, a hydrophobization agent, a hydrophilization agent, a lubricant, a wetting agent and/or a reviving agent.

**4.** Medical or medical-technology article or article for personal protective equipment according to at least one of the aforementioned claims, **characterized in that** the article is antimicrobially effective for at least 2 days, preferably at least 3 days, further preferably at least 4 days, from the first use.

## Revendications

**1.** Article médical ou pour la technique médicale, ou article pour l'équipement de protection personnelle (10), comprenant un matériau de support polymère (12) ou constitué d'un matériau de support polymère (12) avec un premier côté constituant une surface (14) et un deuxième côté constituant une surface (16), qui est muni par le fabricant d'une formulation antimicrobienne, **caractérisé en ce que** la formulation comprend de la polyhexaméthylèneguanidine ou un sel de celle-ci en tant qu'agent antimicrobien, **en ce que** l'article est un gant, en particulier un gant d'examen et/ou un gant chirurgical et/ou un gant de protection, un cathéter, en particulier un cathéter à ballonnet et/ou un cathéter veineux, un composant pour un système de drainage, en particulier d'un système de drainage peropératoire et/ou d'un système de drainage postopératoire, le composant pour un système de drainage comprenant des gants, des tuyaux d'aspiration et les tuyaux de connexion, tous principalement en matériau polymère, une canule ou un tuyau souple à usage dans le domaine médical, un sachet collecteur et/ou un film, en particulier un film de recouvrement/de protection/d'emballage à usage dans le domaine médical, et **en ce que** la formulation antimicrobienne est intégrée dans le matériau de support polymère.

**2.** Article médical ou pour la technique médicale, ou article pour l'équipement de protection personnelle selon la revendication 1, **caractérisé en ce que** la formulation comprend de la polyhexaméthylèneguanidine ou un sel de celle-ci en tant qu'agent antimicrobien ainsi que d'autres auxiliaires ou additifs.

**3.** Article médical ou pour la technique médicale, ou article pour l'équipement de protection personnelle selon la revendication 2, **caractérisé en ce que** la formulation comprend, en tant qu'auxiliaire ou additif, un agent de soin pour la peau, un tensioactif, un agent d'hydrophobisation, un agent d'hydrophilisation, un additif de glissement, un agent réticulant et/ou un agent d'avivage.

**4.** Article médical ou pour la technique médicale, ou article pour l'équipement de protection personnelle selon au moins l'une quelconque des revendications précédentes, **caractérisé en ce que** l'article présente un effet antimicrobien à partir de la première utilisation pendant au moins deux jours, de préférence au moins trois jours, plus préférablement au moins quatre jours.

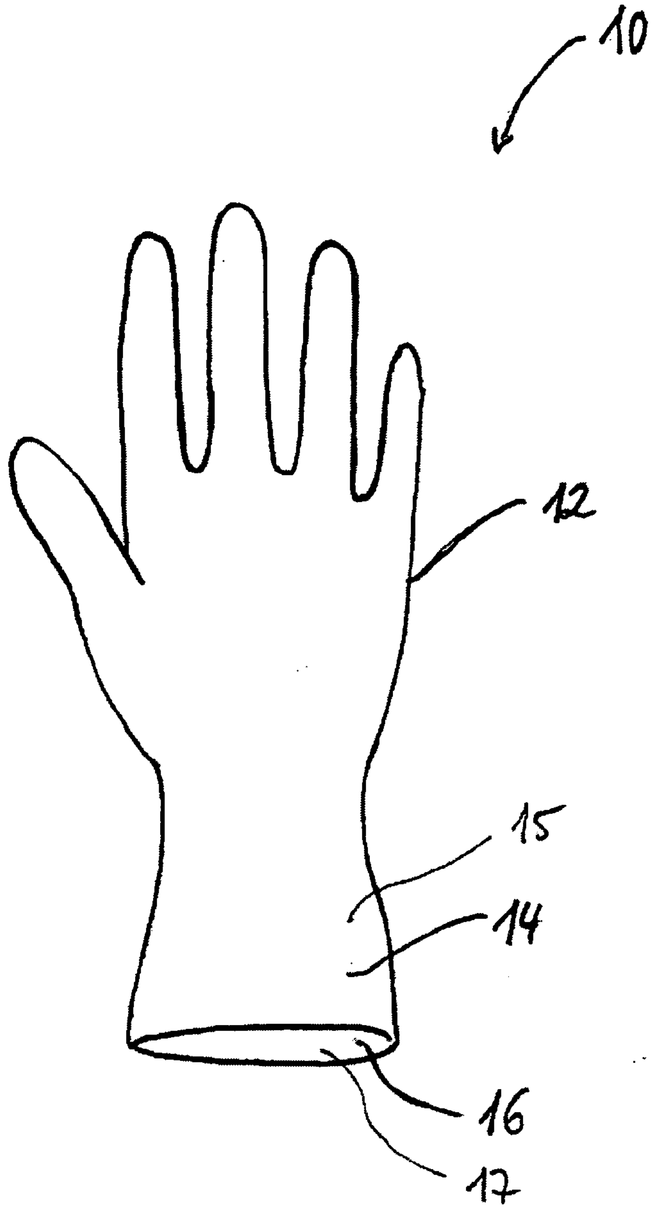


Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009080239 A2
- WO 2007027871 A2


### In der Beschreibung aufgeführte Nicht-Patentliteratur


- **M.K. OULÉ et al.** *Journal of Medical Microbiology,* 2008, vol. 57, 1523-1528
- *CHEMICAL ABSTRACTS,* 57 028-96-3